# EUROPÄISCHE PATENTANMELDUNG

(11) **EP 0 525 444 A1**
(43) Veröffentlichungstag der Anmeldung: **03.02.1993**
(21) Anmeldenummer: 92111460.9
(22) Anmeldetag: 07.07.1992
(51) Int. Cl.: C07C 43/12, C07C 41/30

(54) **Verfahren zur Herstellung von fluorierten Polyethern**

(30) Priorität: 09.07.1991 DE 4122614
(71) Anmelder: HOECHST AKTIENGESELLSCHAFT, D-65926 Frankfurt am Main (DE)
(72) Erfinder: Gries, Thomas, Dr., W-6230 Frankfurt am Main 80 (DE)

(57) **Zusammenfassung**

Die Erfindung betrifft ein Verfahren zur Herstellung von fluorierten Polyethern der Formel I 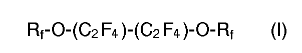
worin R_{f} ein geradkettiger, verzweigter oder cyclischer Fluoralkyl- oder Fluoralkylether-Rest ist und die beiden (C₂F₄)-Gruppen unabhängig voneinander die Struktur (CF₂-CF₂) oder (CF(CF₃)) haben, aus einem Fluorvinylether der Formel II 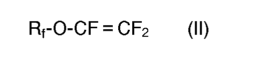
worin R_{f} die gleiche Bedeutung wie in der Formel I hat. Dabei setzt man den flüssigen oder in einem inerten Lösungsmittel gelösten Fluorvinylether (II) mit gasförmigem elementaren Fluor, das unverdünnt oder mit einem Inertgas verdünnt eingesetzt wird, bei einer Temperatur von -80 bis 200 °C um.

## Beschreibung

Die Erfindung betrifft ein Verfahren zur Herstellung von fluorierten Polyethern der Formel I 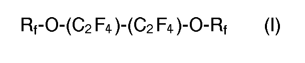
worin R_{f} ein geradkettiger, verzweigter oder cyclischer Fluoralkyl- oder Fluoralkylether-Rest ist und die beiden (C₂F₄)-Gruppen unabhängig voneinander die Struktur (CF₂-CF₂) oder (CF(CF₃)) haben, aus Fluorvinylethern der Formel II 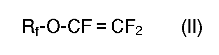
worin R_{f} die gleiche Bedeutung wie in der Formel I hat.

Fluorierte Polyether zeichnen sich durch hohe thermische Beständigkeit, Stabilität gegenüber aggressiven Chemikalien und gute Kompatibilität mit den meisten Werkstoffen aus. Sie werden deshalb bevorzugt als Wärmeträger und Testflüssigkeiten im Elektronikbereich und in der Luft- und Raumfahrttechnik sowie als nichtbrennbare Flüssigkeiten in chemischen Prozessen eingesetzt.

Die Herstellung fluorierter Polyether ist bereits bekannt, z.B. durch Kolbe-Elektrolyse (JP-OS 58 103 334, DE-OS 3 828 848) oder photochemische Decarboxylierung von Perfluorethercarbonsäuren (EP-A-0 298 863), durch Pyrolyse von Perfluoralkoxialkyliodiden (US-PS 3 657 362) sowie durch Reaktion von Perfluorvinylethern mit Perfluoracylfluoriden und photochemische Decarbonylierung der dabei entstandenen perfluorierten Ketone (DE-PS 2 531 511). Die Ausführungsbeispiele in den genannten Literaturstellen zeigen jedoch, daß bei der Kolbe-Elektrolyse wasserstoffhaltige Nebenprodukte entstehen, daß die Pyrolyse von Perfluoralkyliodiden nur eine Ausbeute von höchstens 10% ergibt, und daß die genannten photochemischen Reaktionen nur sehr langsam mit Reaktionszeiten von 16 bis 140 h verlaufen.

Es wurde nun gefunden, daß die Herstellung von fluorierten Polyethern der Formel I direkt aus Fluorvinylethern der Formel II durch Fluorierung mit elementarem Fluor gelingt.

Ein Gegenstand der Erfindung ist ein Verfahren zur Herstellung von fluorierten Polyethern der Formel 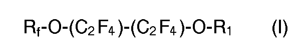
worin R_{f} ein geradkettiger, verzweigter oder cyclischer Fluoralkyl- oder Fluoralkylether-Rest ist und die beiden (C₂F₄)-Gruppen unabhängig voneinander die Struktur (CF₂-CF₂) oder (CF(CF₃)) haben, aus einem Fluorvinylether der Formel II 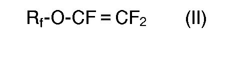
worin R_{f} die gleiche Bedeutung wie in der Formel I hat, dadurch gekennzeichnet, daß man den flüssigen oder in einem inerten Lösungsmittel gelösten Fluorvinylether (11) mit gasförmigem elementarem Fluor, daß unverdünnt oder mit einem Inertgas verdünnt eingesetzt wird, bei einer Temperatur von -80 bis 200 ° C umsetzt.

R_{f} ist bevorzugt ein gesättigter, geradkettiger, verzweigter oder cyclischer Fluoralkyl- oder Fluoralkylether-Rest der Summenformel 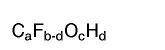
worin a, b, c, d ganze Zahlen sind und
a = 2 bis 30,
b = 4 bis 61,
c = 0 bis 9,
d = 0 bis 4, insbesondere d = 0,
b + d = 2a oder 2a + 1,
b-d≧4,
b/d 2 und
a/c > 2 ist.

Besonders bevorzugt ist R_{f} ein Rest mit der Strukturformel X-CₘF₂ₘ-(OC₃F₆)ₙ, worin x = F oder H, m eine ganze Zahl von 1 bis 3 und n eine ganze Zahl von 0 bis 9 ist, wobei m = 3 für n = 0 gilt. Dabei ist wieder besonders wichtig der Fall, daß X = F ist.

Insbesondere ist R_{f} ein Rest der Strukturformel C₃F₇-(OC₃F₆)p, worin p eine ganze Zahl von 0 bis 8 ist.

Zur Durchführung des erfindungsgemäßen Verfahrens wird der Fluorvinylether der Formel II mit gasförmigem elementarem Fluor, das unverdünnt oder mit einem Inertgas, beispielsweise Stickstoff, Helium oder Argon, verdünnt eingesetzt wird, bei einer Temperatur von -80 bis 200 ° C, bevorzugt von -30 bis 50 °C, insbesondere von -5 bis 30 ° C umgesetzt. Der Fluorvinylether wird bevorzugt ohne Lösungsmittel fluoriert, jedoch kann die Fluorierung - falls gewünscht - auch in einem inerten Lösungsmittel, z.B. einem Perfluorpolyether, erfolgen.

Die Herstellung der Fluorvinylether der Formel II wird in US-PS 2 668 864, US-PS 1 145 445, BE-PS 840 910, Angew. Chem. Int. Ed. Eng. 24 (1985)-,161-179 sowie in Ullmann's Encyclopedia of Industrial Chemistry, Vol. A 11, Fluorine Compounds, Organic, Chapter 6.2 Perfluorovinyl Ethers, S. 366 ff und den dort genannten Literaturstellen beschrieben.

Vorteilhaft wird die Fluorierung in einem Reaktionsgefäß aus einem fluorbeständigem Kunststoff, z.B. FEP, PFA, PCTFE oder PTFE, durchgeführt, wobei das gasförmige Fluor durch eine Gasfritte, beispielsweise aus PTFE, in Form fein verteilter Gasblasen durch den flüssigen oder gelösten Fluorvinylether geleitet wird. In diesen Apparaturen wird ein vollständiger Umsatz des Vinylethers erzielt.

Aus dem nach der Fluorierung vorliegenden Reaktionsgemisch wird gelöstes Fluor - welches bei Fluorierungen immer vorhanden ist - durch Extraktion mit wäßriger Kalilauge entfernt. Der fluorierte Polyether der allgemeinen Formel (I) wird anschließend durch fraktionierte Destillation, bei höhersiedenden Substanzen im Vakuum, in reiner Form abgetrennt.

Das erfindungsgemäße Verfahren besitzt folgende Vorteile:
1. Es wird ein fluorierter Polyether mit definiertem Molekulargewicht erhalten, das mehr als doppelt so groß wie das des eingesetzten Fluorvinylethers ist. Dieser fluorierte Polyether besitzt aufgrund des höheren Molekulargewichts Eigenschaften, die für bestimmte Anwendungen günstig sind, beispielsweise eine höhere Viskosität und einen sehr viel höheren Siedepunkt als ein dem Molekulargewicht des Fluorvinylethers entsprechender fluorierter Ether.
2. Wird ein (wasserstofffreier) Perfluorvinylether eingesetzt, dann entstehen bei dieser Reaktion keine wasserstoffhaltigen organischen Nebenprodukte, da als weiterer Reaktand nur Fluor eingesetzt wird und wasserstoffhaltige Lösungsmittel - die beispielsweise für die erwähnte Kolbe-Elektrolyse notwendig sind - nicht benötigt werden.

Werden zwei verschiedene Fluorvinylether 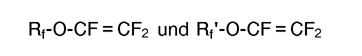
gemeinsam fluoriert, dann entstehen drei verschiedene fluorierte Polyether: 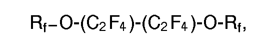 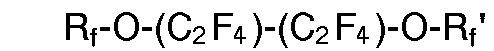
und 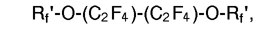
wobei R_{f} und R_{f}' die gleiche Bedeutung wie in der allgemeinen Formel I besitzen. Diese gemeinsame Fluorierung ist ein weiterer Gegenstand der Erfindung.

### Beispiele

Die genannten Prozentangaben bedeuten Gewichtsprozente, sofern nichts anderes erwähnt ist.

### Beispiel 1

Die Fluorierungsapparatur bestand aus einem 1 I-FEP-Gefäß mit einer Gaseinleitungsfritte aus PTFE. Die Fritte war über ein T-Stück mit einer Fluor- und einer Stickstoffdruckgasflasche verbunden. Das Fluorierungsgefäß war mit einem mit Natronkalk gefüllten Fluorabsorber verbunden. Die Messung der Fluor- und Stickstoffmengen erfolgte mit elektronischen Durchflußmeßgeräten der Fa. Hastings. 929 g C₃F₇-0-CF(CF₃)-CF₂-0-CF=CF₂ (2,15 Mol) wurden vorgelegt und mit Eis/Wasser auf 1 bis 3°C gekühlt. 50,2 I Fluor (2,24 Mol) wurden während 15 h eingeleitet, wobei zu Beginn der Reaktion verdünntes Fluor in einer Konzentration von 10 Vol.-% (verdünnt mit Stickstoff) und am Ende reines Fluor verwendet wurde. Während der Reaktion stieg die Temperatur im Reaktionsgefäß auf 22 ° C, da kein weiteres Kühlmittel hinzugefügt wurde. Das Reaktionsgemisch wurde dann zweimal mit jeweils 500 ml 10 %iger wäßriger Kalilauge und einmal mit 500 ml Wasser gewaschen. Es wurden 920 g organische Phase erhalten, aus denen durch fraktionierte Destillation 245 g C₃F₇-(OC₃F₆)-O-(C₂F₄)-(C₂F₄)-O-(C₃F₆O)-C₃F₇ mit einem Siedepunkt von 92 °C bei 10 mbar in einer Reinheit über 99 % isoliert wurden. Die Molekülstruktur der Verbindung wurde durch ¹⁹ F-NMR-Spektroskopie bestimmt.

### Beispiel 2

In der in Beispiel 1 beschriebenen Apparatur wurden 1350 g C₃F₇-O-CF(CF₃)-CF₂-O-CF=CF₂ - (3,12 Mol) vorgelegt und bei einer Temperatur von 10 bis 15°C mit 76,2 I Fluor (3,40 Mol) reagiert. Die Reaktionszeit betrug 14 h. Die Fluorkonzentration betrug zu Beginn des Versuches 40 Vol.-% (Verdünnung mit Stickstoff). Nach 5 h wurde unverdünntes Fluor eingesetzt. Aus den erhaltenen 1364 g Reaktionsprodukt wurden nach Waschen mit wäßriger Kalilauge und fraktionierter Destillation 415 g C₃F₇-(OC₃F₆)-O-(C₂F₄)-(C₂F₄)-O-(C₃F₆O)-C₃ F₇ erhalten.

### Beispiel 3

In der in Beispiel 1 beschriebenen Apparatur wurden 910 g C₃F₇-O-CF(CF₃)-CF₂-O-CF=CF₂ - (2,11 Mol) vorgelegt. Während 6 h wurden 49,5 I Fluor (2,21 Mol) eingeleitet, wobei die Reaktionstemperatur durch Kühlung zwischen 22 und 26°C gehalten wurde. Die Fluorkonzentration betrug zu Beginn des Versuches 30 Vol.-% (verdünnt mit Stickstoff) und wurde nach 3 h auf 100 Vol.-% erhöht. 924 g Produkt wurden erhalten und zur Reinigung mit 400 ml 10 %iger Kalilauge und mit 350 ml Wasser gewaschen. Durch fraktionierte Destillation wurden 296 g C₃F₇-(OC₃F₆)-O-(C₂F₄)-(C₂ F4)-0-(C3 F₆ 0)-C₃ F₇ erhalten.

### Beispiel 4

In einem zylinderförmigen 250 ml-Gefäß aus PTFE wurden 125 g C₃F₇-O-CF=CF₂ (0,47 Mol) vorgelegt und mit Eis/Wasser auf ca. + 1 _{°} C gekühlt. Während 2 h wurden 9 I Fluor (0,4 Mol), verdünnt mit 40 Vol.-% Stickstoff, eingeleitet. Das Reaktionsgemisch wurde danach zweimal mit jeweils 50 ml 10 %iger wäßriger Kalilauge gewaschen und anschließend fraktioniert destilliert. 39,5 g C₃F₇-O-(C₂F₄)-(C₂F₄)-O-C₃F₇ mit einem Siedepunkt von 133°C wurden erhalten. Die Struktur der Substanz wurde mittels ¹⁹F-NMR-Spektroskopie und Infrarotspektroskopie durch Vergleich mit einer Referenzprobe bestimmt.

## Patentansprüche

1. Verfahren zur Herstellung von fluorierten Polyethern der Formel I 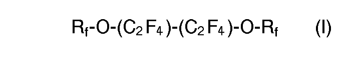
worin R₁ ein geradkettiger, verzweigter oder cyclischer Fluoralkyl- oder Fluoralkylether-Rest ist und die beiden (C₂F₄)-Gruppen unabhängig voneinander die Struktur (CF₂-CF₂) oder (CF-(CF₃)) haben, aus einem Fluorvinylether der Formel II 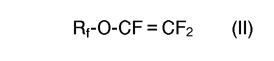
worin R_{f} die gleiche Bedeutung wie in der Formel I hat, dadurch gekennzeichnet, daß man den flüssigen oder in einem inerten Lösungsmittel gelösten Fluorvinylether (II) mit gasförmigem elementaren Fluor, das unverdünnt oder mit einem Inertgas verdünnt eingesetzt wird, bei einer Temperatur von -80 bis 200 ° C umsetzt.

2. Verfahren nach Anspruch 1, dadurch gekennzeichnet, daß der Rest R_{f} ein gesättigter, geradkettiger, verzweigter oder cyclischer Fluoralkyl- oder Fluoralkylether-Rest der Summenformel 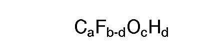
ist, worin a, b, c, d ganze Zahlen sind und a = 2 bis 30,
b = 4 bis 61,
c = 0 bis 9,
d = 0 bis 4, insbesondere d = 0,
b + d = 2a oder 2a + 1,
b-d≧4,
b/d 2 und
a/c > 2 ist.

3. Verfahren nach Anspruch 1, dadurch gekennzeichnet, daß der Rest R_{f} die Strukturformel 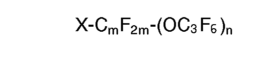
hat, worin
X = F oder H, insbesondere X = F,
m eine ganze Zahl von 1 bis 3 und
n eine ganze Zahl von 0 bis 9 ist,
wobei m = 3 für n = 0 gilt.

4. Verfahren nach Anspruch 1, dadurch gekennzeichnet, daß der Rest R_{f} die Strukturformel C₃F₇-(OC₃F₆)ₚ hat, worin p eine ganze Zahl von 0 bis 8 ist.

5. Verfahren zur Herstellung eines Gemisches von Fluorpolyethern der Formeln I, I' und I" 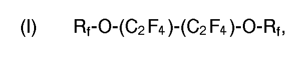 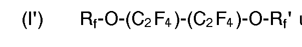
and 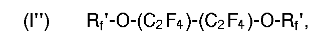
worin R_{f} und R_{f}' voneinander verschiedene, geradkettige, verzweigte oder cyclische Fluoralkyl- oder Fluoralkylether-Reste sind und die (C₂F₄)-Gruppen unabhängig voneinander die Struktur (CF₂-CF₂) oder (CF(CF₃)) haben, aus einem Gemisch der Fluorvinylether der Formeln II und II' 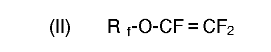 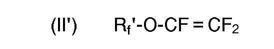
worin R_{f} und R_{f}' dieselbe Bedeutung wie in den Formeln I, I' und I" haben, dadurch gekennzeichnet, daß man das flüssige oder in einem inerten Lösungsmittel gelöste Gemisch der Fluorvinylether II und II' mit gasförmigen elementarem Fluor, das unverdünnt oder mit einem Inertgas verdünnt eingesetzt wird, bei einer Temperatur von -80 bis 200 ° C umsetzt.

6. Verfahren nach Anspruch 5, dadurch gekennzeichnet, daß die Reste R_{f} und R_{f}' voneinander verschiedene gesättigte, geradkettige, verzweigte oder cyclische Fluoralkyl- oder Fluoralkylether-Reste der Summenformel 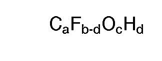
sind, worin a, b, c, d ganze Zahlen sind und
a = 2 bis 30,
b = 4 bis 61,
c = 0 bis 9,
d = 0 bis 4, insbesondere d = 0,
b + d = 2a oder 2a + 1,
b-d≧4,
b/d ≧ 2 und
a/c > 2 ist.

7. Verfahren nach Anspruch 5, dadurch gekennzeichnet, daß die Reste R_{f} und R_{f}' voneinander verschieden sind und die Strukturformel 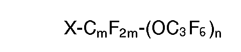
haben, worin
X = F oder H, insbesondere X = F,
m eine ganze Zahl von 1 bis 3 und
n eine ganze Zahl von 0 bis 9 ist,
wobei m = 3 für n = 0 gilt.

8. Verfahren nach Anspruch 5, dadurch gekennzeichnet, daß die Reste R_{f} und R_{f}' voneinander verschieden sind und die Strukturformel C₃F₇-(OC₃F₆)p haben, worin p eine ganze Zahl von 0 bis 8 ist.

9. Verfahren nach einem der Ansprüche 1 bis 8, dadurch gekennzeichnet, daß man die Umsetzung bei -30 bis 50 °C durchführt.

10. Verfahren nach einem der Ansprüche 1 bis 8, dadurch gekennzeichnet, daß man die Umsetzung bei -5 bis 30 °C durchführt.

11. Verfahren nach einem der Ansprüche 1 bis 10, dadurch gekennzeichnet, daß man mit Stickstoff, Helium oder Argon verdünntes Fluor einsetzt.
